# EUROPEAN PATENT SPECIFICATION

(11) **EP 4 271 428 B1**
(45) Date of publication and mention of the grant of the patent: **13.05.2026**
(21) Application number: 21916065.2
(22) Date of filing: 28.12.2021
(51) Int. Cl.: A61L 27/50, A61L 27/52, A61P 19/02, C08L 5/08

(54) **INJECTABLE PHOTOCROSSLINKED HYALURONIC ACID HYDROGELS, PRODUCTION METHOD THEREOF AND THEIR USE FOR THE TREATMENT OF OSTEOARTHRITIS**
INJIZIERBARE PHOTOVERNETZTE HYALURONSÄUREHYDROGELE, HERSTELLUNGSVERFAHREN DAFÜR UND DEREN VERWENDUNG ZUR BEHANDLUNG VON OSTEOARTHRITIS
HYDROGELS INJECTABLES D'ACIDE HYALURONIQUE PHOTORÉTICULÉ, LEUR PROCÉDÉ DE PRODUCTION ET LEUR UTILISATION DANS LE TRAITEMENT DE L'ARTHROSE

(30) Priority: 29.12.2020 TR 202022151
(43) Date of publication of application: 08.11.2023
(73) Proprietor: VSY Biyoteknoloji Ve Ilac Sanayi Anonim Sirketi, Tuzla/Istanbul (TR)
(72) Inventor: OYTUN, Faruk, Tuzla/Istanbul (TR); ÇIFTÇI, Mustafa, Yildirim/Bursa (TR); KAYA, Busra Gizem, Tuzla/Istanbul (TR)
(74) Representative: Sevinç, Cenk
(86) International application number: PCT/TR2021/051565
(87) International publication number: WO 2022/146387

(56) References cited:
- CN-A- 112 062 981
- JP-B2- 5 607 061
- KR-A- 20070 073 008
- US-B2- 8 242 179
- SIGEN A ET AL: "A facile one-pot synthesis of acrylated hyaluronic acid", vol. 54, no. 9, 1 January 2018 (2018-01-01), UK, pages 1081 - 1084, XP093212445, ISSN: 1359-7345, Retrieved from the Internet <URL:https://pubs.rsc.org/en/content/articlepdf/2018/cc/c7cc08648b> DOI: 10.1039/C7CC08648B
- MATSUDA TAKEHISA ET AL.: "Preparation of vinylated polysaccharides and photofabrication of tubular scaffolds as potential use in tissue engineering", BIOMACROMOLECULES, vol. 3, no. 5, 2002, pages 942 - 50, XP002414698, DOI: 10.1021/bm0200229
- HADI SAMADIAN ET AL.: "Natural polymers-based light-induced hydrogels: Promising biomaterials for biomedical applications", COORDINATION CHEMISTRY REVIEWS, vol. 420, 2020, pages 213432, XP086220689, DOI: https://doi.org/10.1016/j.ccr.2020.213432

## Description

### Field of the Invention

The present invention relates to the development of an injectable viscoelastic hydrogel formulation comprising photocrosslinked hyaluronic acid in order to relieve pain caused by osteoarthritis (OA), to support synovial fluid which has decreased activity, and to treat osteochondral lesions.

### Background of the Invention

The *joint* can be defined as the location where two bones come together in movable parts of the body such as arms, legs, feet, wrists, and the ends of these bones are covered with a protective tissue called cartilage. Cartilage is a durable and slippery tissue which allows joint movement to occur almost without friction. Synovial fluid, which provides lubricity between bone ends and cartilage, is comprised of Hyaluronic Acid (HA) that is naturally present in the body. The viscosity, lubrication and shock absorbing properties of the said synovial fluid decrease over time due to mechanical effects and the cartilages begin to rub against each other and break down. As the cartilage wears off and breaks down over time, the friction between the bones increases and a joint disease called Osteoarthritis (OA) begins and progresses with the increase in friction.

*Osteoarthritis* (OA) is the most common, chronic (long-term) joint disease affecting millions of people worldwide. OA is also called degenerative joint disease, degenerative arthritis, and wear and tear arthritis. OA affects the entire joint, as well as cartilage breakdown. It causes changes in the bone structure and the deterioration of the connective tissues that hold the joint together and connect the muscles to the bone; and it also causes inflammation of the joint capsule. Even though OA is usually seen in old ages, it can sometimes be seen in young people.

OA symptoms develop over time and they worsen as it progresses. Symptoms of OA include pain, stiffness, tenderness, loss of flexibility, crepitus, osteophyte formation, and swelling.

Even though damage of the joints cannot be recovered; exercising, being at a healthy weight and several treatments that are applied can slow the progression of the disease and help relieve pain and improve joint functions.

One of the methods that can help relieve OA symptoms, especially pain, is the use of medication. The commonly used drugs and their effects are shown below:
- **Acetaminophen:** It is known that acetaminophen (Tylenol and others) helps patients with osteoarthritis suffering from mild to moderate pain. Taking acetaminophen more than the recommended dose may cause liver damage.
- **Non-steroidal anti-inflammatory drugs (NSAIDs):** NSAIDs such as Advil (ibuprofen) and Aleve (naproxen) reduce swelling as well as pain.
- **Duloxetine (Cymbalta):** This drug which is generally used as an antidepressant is also used to treat many chronic pains, including osteoarthritis pain.

In addition to these, surgical and other procedures that can be used to relieve the symptoms of OA are listed below:
- **Cortisone injections:** In corticosteroid drug injections, the area around the joint is checked, and then the drug is injected by inserting a needle into the space in the joint. However, the number of cortisone injections that can be administered annually is generally limited to three or four. This is because the drug worsens joint damage over time.
- **Viscoelastic injections:** Injections of HA, which is a component found naturally in the synovial fluid, exhibit a buffering effect on the knee and alleviate the severity of pain.
- **Joint replacement:** In joint replacement surgery (arthroplasty), the damaged joint surfaces are replaced with plastic and metal parts. However, this method involves surgical risks related with the infections. In addition, artificial joints can also wear out and loosen over time and may need to be replaced.

Today, the most preferred method among the methods of the treatment is to make viscoelastic injections to the problematic area. The most widely used of these injection types is HA injections. People with OA have low amounts of HA in their joint fluid. In the knee with OA, the concentration of HA is approximately half of the normal level. HA is a gel-like substance naturally existing in the synovial fluid surrounding the joints. The said substance provides lubrication and cushioning, acts as a shock absorber, and helps the bones move smoothly by preventing the bones from wearing each other off. Furthermore, it supports the synovial fluid to ensure the healing of intra-articular wounds in pathological conditions. In addition, it improves the physiological environment of the osteoarthritic joint and increases its mobility by regaining its viscoelasticity.

Changes in the viscous and elastic properties of HA decrease the joint's ability to withstand tensile and shear forces. HA injections can help recover the normal levels of synovial fluid. These injections not only lubricate the joint, they can also reduce inflammation and protect the cartilage from further wear and tear.

The injections are made in weekly periods by the doctor or healthcare professional. The number of injections to be made may vary depending on the HA concentration. Before making the injection, the doctor can perform aspiration by taking some of the fluid from the knee joint with a needle in order to reduce swelling.

The hyaluronic acid which is not crosslinked (Linear HA) has several disadvantages such as its short term effect and its inability to create volume. HA is subjected to crosslinking processes with crosslinking agents such as ethyldimethylaminopropyl carbodiimide (EDC), butanediol diglycidylether (BDDE), glutaraldehyde (GTA) or divinyl sulfone (DVS) in order to improve its viscoelastic properties and increase its permanence. Crosslinking reaction is an intramolecular and intermolecular esterification reaction that is performed using several reagents. It has been envisaged in this technique, the residence time of the HA formulation in the joint can be increased. Therefore, while linear HA injections are usually applied in 3-5 sessions, this process can be performed in a single session in viscoelastics comprising cross-linked HA. This makes cross-linked HA injections, which have a longer residence time in the body and higher mechanical strength, to be preferred more.

The physical, chemical and biological properties of HA change significantly by increasing the degree of crosslinking. The gel suspension of crosslinked HA has different rheological properties than the linear HA. The rheological properties of cross-linked HA gels exhibit superior properties than non-crosslinked HA at the same concentration.

In the state of the art, in cases when HA is applied to the joint area, it changes the rheology of the synovial fluid, provides a quick relief in mobility and relieves pain. However, the effect of the conventionally used Linear HA is temporary, because HA can only stay for 72 hours within the joint before it is absorbed or metabolized. As well as this situation does not prolong the patient's comfort period sufficiently, the damaged articular cartilage cannot be healed since the main problem is not improved.

United States patent application no US8323617B2, an application known in the state of the art, discloses that HA formulations crosslinked with carbodiimide derivative crosslinking agents are used in the treatment of OA. Since the formulations obtained by the direct crosslinking method require harsh reaction conditions, there is a possibility that toxic by-products may be generated; and since the crosslinking agents that are used are cytotoxic, they may not be considered suitable for hydrogel design.

European patent document no EP1443945B1, an application known in the state of the art, discloses synergistic effect of sodium hyaluronate and chondroitin sulfate mixture on the lubrication and regeneration of articular cartilage damaged by stage I and stage II osteoarthritis of human joints. In this patent application, the combination of linear HA and chondroitin sulfate was used in the treatment of osteoarthritis. It is believed that it is unlikely that the viscoelastic product to be used in such formulations where crosslinking agent is not used will provide comfort to the patient in a single session.

In the patent document US8242179B2, hyaluronic acid-based hydrogel formulations used for the treatment of osteoarthritis are obtained by direct crosslinking with carbodiimide-derived synthetic crosslinkers. However, this method results in the formation of toxic by-products due to the high reaction intensity and the need for long and laborious purification processes to remove crosslinker residues that pose a problem in terms of biocompatibility. Furthermore, the cytotoxic effects of the crosslinkers used limit the safety and biocompatibility of the application. The traditional method described in D1 also fails to meet the advantages of modern production, such as low energy consumption, ambient temperature, solvent-free environment and temporal/spatial control.

An article published by Falcone, S. J. et. al., an application known in the state of the art, discloses the use of hyaluronic acid in biomedical applications, its physical properties, rheological properties and differences of cross-linked HA from linear HA. **[1]**

### Summary of the Invention

The objective of the present invention is to develop an injectable viscoelastic hydrogel formulation comprising photocrosslinked hyaluronic acid in order to relieve pain caused by osteoarthritis (OA), to support synovial fluid which has decreased activity, and to treat osteochondral lesions.

Another objective of the invention is to significantly reduce the purification processes that are required for the removal of synthetic crosslinkers, which can exhibit toxic properties, from the formulation and that require long periods in the production process.

A further objective of the invention is to not use synthetic crosslinkers with toxic effects, but to carry out the crosslinking process through camphorquinone, which is widely used in many bio-applications, especially in dental applications.

Yet another objective of the present invention is to adapt photo-initiated polymerization methods, which meet many economic and ecological expectations, to the production of hyaluronic acid hydrogels.

### Detailed Description of the Invention

**"Injectable Photocrosslinked Hyaluronic Acid Hydrogels and Their Use for the Treatment of Osteoarthritis"** developed in order to fulfil the objectives of the present invention is illustrated in the accompanying figures, in which:
**Figure 1****-** Illustration of the reaction of obtaining initiator radicals using second type of photo-initiators. (**A:** Illustration of general reaction, **B**: Benzophenone example).
**Figure 2****-** Illustration of the synthesis of acrylated hyaluronic acid monomer in single step.
**Figure 3****-** Illustration of the production of HA-based hydrogels via camphorquinone with the effect of light without crosslinker.

The components shown in the figures are each given reference numbers as follows:
**FB.** Photo-initiator
**[FB]*.** Excited state photo-initiator
**YB.** Co-initiator
**YB^{•}.** Activated co-initiator
**hv.** Light
**TEA.** Triethylamine
**HA-TH.** Hyaluronic Acid Based Hydrogel

A production method of an injectable viscoelastic hydrogel formulation comprising photocrosslinked hyaluronic acid in order to relieve the pain caused by osteoarthritis (OA) and to support synovial fluid which has decreased activity comprises the steps of
- Production of acrylated hyaluronic acid monomer,
   o Dissolving hyaluronic acid (HA) by slowly adding it into deionized water,
   o Dissolving glycidyl acrylate and triethylamine in dimethylformamide,
   o Combining these two solutions and mixing for 3 days at room temperature,
   o Precipitating the mixture in acetone weighing up to 20 times its own weight and filtering it under low pressure,
   o Removing the impurities of the obtained solid with deionized water,
   o Drying the mixture by freeze drying method,
   o Obtaining acrylated HA monomer,
- Production of photocrosslinked hyaluronic acid hydrogels,
   o Dissolving the obtained acrylated HA monomer by slowly adding it into deionized water,
   o Adding camphorquinone to this HA mixture and mixing it homogeneously,
   o Illuminating the mixture under visible light and leaving it for crosslinking reaction,
   o Neutralizing the gel in the buffer solution after the reaction until the pH value becomes stable,
   o Filtering after pH adjustment, and allowing the gel to swell upon adding a new buffer solution,
   o Then, bringing the obtained gel to a particle size of 50-300 microns,
   o Adding non-crosslinked HA (Linear HA) into the photocrosslinked HA gel to facilitate its extrusion,
   o Adjusting the pH of the final mixture to 7 and filling it into the syringes by applying vacuum,
   o Applying vapor sterilization process to the syringes after filling process.

In one embodiment of the invention, 5.0 mmol (2.0 grams) of HA is slowly added into 100 mL of deionized water and dissolved therein within the scope of production of acrylated hyaluronic acid monomer.

In one embodiment of the invention, 250 mmol (32.2 grams) of glycidyl acrylate and 250 mmol (25.3 grams) of triethylamine are dissolved in 100 mL of dimethylformamide.

In one embodiment of the invention, 5.0 mmol (2.0 g) of acrylated HA monomer is slowly added into 100 ml of deionized water and dissolved therein within the scope of production of photocrosslinked HA hydrogels.

In one embodiment of the invention, 0.05-1 % by weight of camphorquinone is added to the HA mixture and mixed homogenously.

In one embodiment of the invention, the mixture of camphorquinone and HA is illuminated under visible light for 30-240 minutes at room temperature and left for crosslinking reaction.

In one embodiment of the invention, after the crosslinking reaction, the gel is neutralized with 0.1 M of hydrochloric acid (HCl) in phosphate buffer solution (PBS) until the pH is 6.8-7.4.

The viscoelastic gel formulation obtained within the scope of the invention is used for the treatment of osteochondral lesions caused by osteoarthritis (OA). The viscoelastic gel formulation obtained within the scope of the invention is used in intra-articular injection applications at concentrations of 10-25 mg/ml.

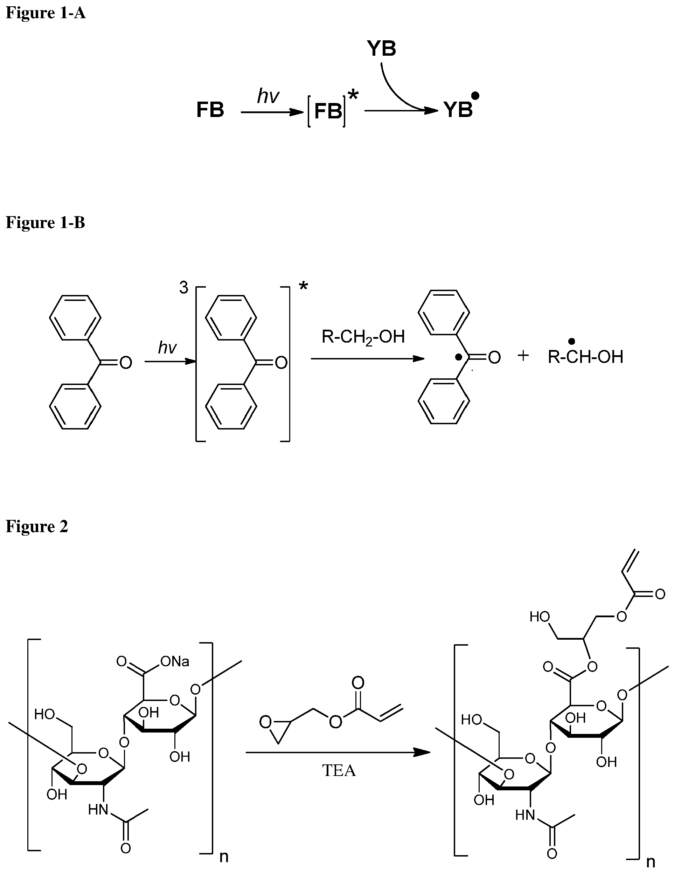

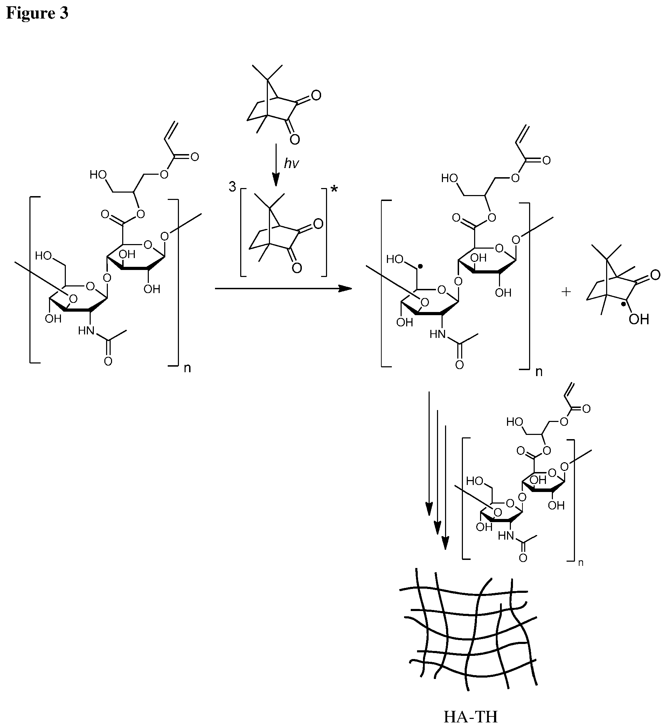

Within the scope of the invention, initially, the hyaluronic acid macromonomer comprising acrylate groups is synthesized in a simple way in a single step (Figure 2). When the obtained acrylated hyaluronic acid monomer and camphorquinone are dissolved in water and illuminated under visible light, HA-based hydrogels are obtained (Figure 3). In the first step of the process, the camphorquinone is excited with the effect of light and then the excited camphorquinone forms initiator radicals by abstracting a hydrogen atom (α hydrogen) from the carbon atom (α carbon) neighboring the hydroxyl group on the hyaluronic acid. These radicals that are formed initiate the free radical polymerization of acrylate groups in the HA monomer, enabling the formation of HA-based hydrogels. Since the said HA monomers comprise more than one acrylate group, gels are obtained easily without any crosslinkers. In other words, the acrylated HA monomer acts both as a conventional monomer and as a crosslinker. In addition, it is predicted that gels with different crosslinking densities can be obtained by changing the camphorquinone concentration. Therefore, gels with different crosslink densities that are needed can be obtained in a simple way.

The viscoelastic gel formulation developed within the scope of the invention, unlike conventional methods, enables to obtain photocrosslinked production of hydrogels comprising HA without any crosslinkers. Acrylated hyaluronic acid and camphorquinone, which are the two main components of the formulation, can be easily obtained as a result of illumination under visible light. Therefore, the need for crosslinking agents such as *EDC* (1-ethyl-3-(3-dimethylaminopropyl)carbodiimide hydrochloride), *BDDE* (1,4-butanediol diglycidyl ether), *GTA* (glutaraldehyde), and *DVS* (divinyl sulfone) in conventional applications is eliminated. As a result of this, the purification processes that require long time and effort, namely the removal processes of these crosslinkers that are used from the final product, will be significantly reduced. For example, the amount of crosslinker BDDE must be less than 2 ppm in the final product, as per the requirements set by the FDA. This may lead to the utilization of multiple washing processes and special purification methods in the purification step. On the other hand, in the method used in the development of the viscoelastic gel formulation developed within the scope of the invention, such crosslinkers with toxic effects will not be used, and the crosslinking will be carried out through camphorquinone, which is widely used in many bio-applications, especially in dental applications.

***Camphorquinone*** is a second type photo-initiator which absorbs visible light between 400-500 nm (maximum 468 nm). In general, in the second type of photo-initiators, the initiator becomes excited by the effect of light. However, since the excitation energies of these excited states are lower than the bond dissociation energies, they cannot directly form initiator radicals. Instead, excited types transfer energy with a second type called co-initiator, abstract hydrogen from co-initiators and generate radicals on the co-initiators (Figure 1). In the second type photo-initiators, alcohol, amine, ether and thiol molecules, which have hydrogen donor properties, are used as co-initiators.

Even though there are different types of second type photo-initiators in the state of the art, camphorquinone has a special importance among these initiators as it exhibits many superior properties. Since camphorquinone can be excited in the visible region of the light (low energy), it both provides energy saving and it is suitable for biological applications since UV region light having harmful effects is not used. Hence camphorquinone is a second type photo-initiator which is most widely used in many bio-applications, especially in dental filling materials. The most important feature of camphorquinone that makes it widely used in such biological applications is its biocompatibility. In addition, camphorquinone shows negative effects in the body only at high concentrations [2, 3]. Furthermore, due to the nature of the reaction, since camphorquinone will not bind to the gel structure that will be formed, it can be easily removed. This will considerably shorten the purification process.

Within the scope of the invention, the photo-initiated polymerization method, which is a method that meets many economic and ecological expectations, is adapted to the production of hyaluronic acid hydrogels. Therefore, the advantages of photo-initiated polymerization methods such as high polymerization rate at low temperatures (including room temperature), low energy consumption, polymerization in solvent-free environment, and temporal/spatial control facilitate the use of this technique in industrial production.

The advantages of the production method of gel formulation according to the present invention can be listed as follows;
- Easily obtaining hydrogels by means of illuminating the two main components of the formulation, namely acrylated hyaluronic acid and camphorquinone, under visible light,
- Obtaining hydrogels comprising HA in a photocrosslinked form without using any synthetic crosslinkers unlike conventional methods,
- Significantly reducing the purification processes that are required for the removal of synthetic crosslinkers which can exhibit toxic properties from the formulation and that require long periods in the production process.
- Facilitating the use of this technique in industrial production with the advantages of photo-initiated polymerization methods such as high polymerization rate at low temperatures (including room temperature), low energy consumption, polymerization in solvent-free environment, and temporal/spatial control by means of adapting photo-initiated polymerization methods to the production of hyaluronic acid hydrogels,
- Increasing the mechanical strength of the gel and its residence time in the joint by means of the photocrosslinked HA in the mixture.

### REFERENCES

**[1].** Falcone, S. J., Palmeri, D., & Berg, R. A. (2006): "Biomedical Applications of Hyaluronic Acid"; Polysaccharides for Drug Delivery and Pharmaceutical Applications, 155-174. doi:10.1021/bk-2006-0934.ch008.
**[2].** M. C. Chang, L. D. Lin, M. T. Wu et al., Effects of camphorquinone on cytotoxicity, cell cycle regulation and prostaglandin E2 production of dental pulp cells: role of ROS, ATM/ Chk2, MEK/ERK and hemeoxygenase-1, PLoS One, vol. 10, no. 12, article e0143663, 2015.
**[3].** Geurtsen W. Biocompatibility of resin-modified filling materials. Crit Rev Oral Biol Med 2000;11(3):333-55.

## Claims

1. A production method of an injectable viscoelastic hydrogel formulation comprising photocrosslinked hyaluronic acid in order to relieve the pain caused by osteoarthritis (OA) and to support synovial fluid which has decreased activity comprising the steps of
- Production of acrylated hyaluronic acid monomer,
o Dissolving hyaluronic acid (HA) by slowly adding it into deionized water,
o Dissolving glycidyl acrylate and triethylamine in dimethylformamide,
o Combining these two solutions and mixing thereof for 3 days at room temperature,
o Precipitating the mixture in acetone weighing up to 20 times its own weight and filtering it under low pressure,
o Removing the impurities of the obtained solid with deionized water,
o Drying the mixture by freeze drying method,
o Obtaining acrylated HA monomer,
- Production of photocrosslinked hyaluronic acid hydrogels,
o Dissolving the obtained acrylated HA monomer by slowly adding it into deionized water,
o Adding camphorquinone to this HA mixture and mixing it homogeneously,
o Illuminating the mixture under visible light and leaving it for crosslinking reaction,
o Neutralizing the gel in the buffer solution after the reaction until the pH value becomes stable,
o filtering after pH adjustment, and allowing the gel to swell upon adding a new buffer solution,
o Then, bringing the obtained gel to a particle size of 50-300 microns,
o Adding non-crosslinked HA (Linear HA) into the photocrosslinked HA gel to facilitate its extrusion,
o Adjusting the pH of the final mixture to 7 and filling it into the syringes by applying vacuum,
o Applying vapor sterilization process to the syringes after filling process.

2. A production method of an injectable viscoelastic hydrogel formulation comprising photocrosslinked hyaluronic acid according to claim 1, wherein 5.0 mmol (2.0 grams) of HA is slowly added into 100 mL of deionized water and dissolved therein within the scope of production of acrylated hyaluronic acid monomer.

3. A production method of an injectable viscoelastic hydrogel formulation comprising photocrosslinked hyaluronic acid according to claim 1, wherein 250 mmol (32.2 grams) of glycidyl acrylate and 250 mmol (25.3 grams) of triethylamine are dissolved in 100 mL of dimethylformamide.

4. A production method of an injectable viscoelastic hydrogel formulation comprising photocrosslinked hyaluronic acid according to claim 1, wherein 5.0 mmol (2.0 g) of acrylated HA monomer is slowly added into 100 ml of deionized water and dissolved therein within the scope of production of photocrosslinked HA hydrogels.

5. A production method of an injectable viscoelastic hydrogel formulation comprising photocrosslinked hyaluronic acid according to claim 1, wherein 0.05-1 % by weight of camphorquinone is added to the HA mixture and mixed homogenously.

6. A production method of an injectable viscoelastic hydrogel formulation comprising photocrosslinked hyaluronic acid according to claim 1, wherein the mixture of camphorquinone and HA is illuminated under visible light for 30-240 minutes at room temperature and left for crosslinking reaction.

7. A production method of an injectable viscoelastic hydrogel formulation comprising photocrosslinked hyaluronic acid according to claim 1, wherein the gel is neutralized after the crosslinking reaction with 0.1 M of hydrochloric acid (HCl) in phosphate buffer solution (PBS) until the pH is 6.8-7.4.

## Patentansprüche

1. Verfahren zur Herstellung einer injizierbaren viskoelastischen Hydrogelformulierung, die lichtvernetzte Hyaluronsäure enthält, um die durch Osteoarthritis (OA) verursachten Schmerzen zu lindern und die Synovialflüssigkeit, die eine verminderte Aktivität aufweist, zu unterstützen, umfassend die Schritte
- Herstellung von acryliertem Hyaluronsäuremonomer,
o Auflösen von Hyaluronsäure (HA) durch langsame Zugabe in deionisiertem Wasser,
o Auflösen von Glycidylacrylat und Triethylamin in Dimethylformamid,
o Kombinieren dieser beiden Lösungen und Mischen für 3 Tage bei Raumtemperatur,
o Ausfällen des Gemischs in Aceton bis zum 20-fachen seines Eigengewichts und Abfiltrieren bei niedrigem Druck,
o Entfernen der Verunreinigungen des erhaltenen Feststoffs mit entionisiertem Wasser,
o Trocknen des Gemischs durch Gefriertrocknung,
o Gewinnung von acryliertem HA-Monomer,
- Herstellung von photovernetzten Hyaluronsäure-Hydrogelen,
o Lösen des erhaltenen acrylierten HA-Monomers durch langsame Zugabe in deionisiertem Wasser,
o Zugabe von Campherchinon zu dieser HA-Mischung und homogenes Mischen,
o Bestrahlung der Mischung mit sichtbarem Licht und Vernetzungsreaktion,
o Neutralisieren des Gels in der Pufferlösung nach der Reaktion, bis der pH-Wert stabil wird,
o Filtrieren nach der pH-Einstellung und Aufquellenlassen des Gels bei Zugabe einer neuen Pufferlösung,
o Anschließend wird das erhaltene Gel auf eine Partikelgröße von 50-300 Mikrometern gebracht,
o Zugabe von unvernetztem HA (lineares HA) in das photovernetzte HAGel, um dessen Extrusion zu erleichtern,
o Einstellen des pH-Werts der endgültigen Mischung auf 7 und Abfüllen in die Spritzen durch Anlegen von Vakuum,
o Anwendung eines Dampfsterilisationsverfahrens für die Spritzen nach dem Abfüllvorgang.

2. Verfahren zur Herstellung einer injizierbaren viskoelastischen Hydrogelformulierung, die photovernetzte Hyaluronsäure gemäß Anspruch 1 umfasst, wobei 5,0 mmol (2,0 Gramm) HA langsam in 100 ml entionisiertes Wasser gegeben und darin im Rahmen der Herstellung von acryliertem Hyaluronsäuremonomer gelöst wird.

3. Verfahren zur Herstellung einer injizierbaren viskoelastischen Hydrogelformulierung, die photovernetzte Hyaluronsäure gemäß Anspruch 1 umfasst, wobei 250 mmol (32,2 g) Glycidylacrylat und 250 mmol (25,3 g) Triethylamin in 100 ml Dimethylformamid gelöst werden.

4. Verfahren zur Herstellung einer injizierbaren viskoelastischen Hydrogelformulierung, die photovernetzte Hyaluronsäure gemäß Anspruch 1 umfasst, wobei 5,0 mmol (2,0 g) acryliertes HA-Monomer langsam in 100 ml entionisiertes Wasser gegeben und darin im Rahmen der Herstellung von photovernetzten HA-Hydrogelen gelöst wird.

5. Verfahren zur Herstellung einer injizierbaren viskoelastischen Hydrogelformulierung, die photovernetzte Hyaluronsäure gemäß Anspruch 1 umfasst, wobei 0,05-1 Gew.-% Campherchinon zu der HA-Mischung gegeben und homogen gemischt wird.

6. Verfahren zur Herstellung einer injizierbaren viskoelastischen Hydrogelformulierung, die photovernetzte Hyaluronsäure gemäß Anspruch 1 umfasst, wobei das Gemisch aus Campherchinon und HA 30-240 Minuten lang bei Raumtemperatur mit sichtbarem Licht beleuchtet und für die Vernetzungsreaktion belassen wird.

7. Verfahren zur Herstellung einer injizierbaren viskoelastischen Hydrogelformulierung, die photovernetzte Hyaluronsäure gemäß Anspruch 1 umfasst, wobei das Gel nach der Vernetzungsreaktion mit 0,1 M Salzsäure (HC1) in Phosphatpufferlösung (PBS) neutralisiert wird, bis der pH-Wert 6,8-7,4 beträgt.

## Revendications

1. Procédé de fabrication d'une formulation d'hydrogel viscoélastique injectable comprenant de l'acide hyaluronique photoréticulé afin de soulager la douleur causée par l'arthrose (OA) et de soutenir le liquide synovial dont l'activité a diminué, comprenant les étapes suivantes :
- Production de monomère d'acide hyaluronique acrylé,
o Dissolution de l'acide hyaluronique (HA) en l'ajoutant lentement dans de l'eau déionisée,
o Dissolution d'acrylate de glycidyle et de triéthylamine dans du diméthylformamide,
o Combination de ces deux solutions et leur mélange pendant 3 jours à température ambiante,
o Précipitation du mélange dans de l'acétone pesant jusqu'à 20 fois son propre poids et sa filtration sous basse pression,
o Élimination des impuretés du solide obtenu à l'aide d'eau déionisée,
o Séchage du mélange par lyophilisation,
o Obtention d'un monomère d'AH acrylé,
- Production d'hydrogels d'acide hyaluronique photoréticulés,
o Dissolution du monomère HA acrylé obtenu en l'ajoutant lentement dans de l'eau déionisée,
o Ajout de la camphoquinone à ce mélange HA et son mélange de manière homogène,
o Illumination du mélange sous lumière visible et son maintien pour permettre la réaction de réticulation,
o Neutralisation du gel dans la solution tampon après la réaction jusqu'à ce que le pH se stabilise,
o Filtration après ajustement du pH et maintien du gel de manière gonflé après ajout d'une nouvelle solution tampon,
o Ensuite, ramener le gel obtenu à une taille de particules comprise entre 50 et 300 microns,
o Ajout de l'AH non réticulé (AH linéaire) au gel d'AH photoréticulé afin de faciliter son extrusion,
o Ajustement du pH du mélange final à 7 et remplissage des seringues sous vide,
o Application du processus de stérilisation à la vapeur aux seringues après le processus de remplissage.

2. Procédé de fabrication d'une formulation d'hydrogel viscoélastique injectable comprenant de l'acide hyaluronique photoréticulé selon la revendication 1, dans lequel 5,0 mmol (2,0 grammes) d'AH sont lentement ajoutés à 100 ml d'eau déionisée et dissous dans celle-ci dans le cadre de la production de monomère d'acide hyaluronique acrylé.

3. Procédé de fabrication d'une formulation d'hydrogel viscoélastique injectable comprenant de l'acide hyaluronique photoréticulé selon la revendication 1, dans lequel 250 mmol (32,2 grammes) d'acrylate de glycidyle et 250 mmol (25,3 grammes) de triéthylamine sont dissous dans 100 ml de diméthylformamide.

4. Procédé de fabrication d'une formulation d'hydrogel viscoélastique injectable comprenant de l'acide hyaluronique photoréticulé selon la revendication 1, dans lequel 5,0 mmol (2,0 g) de monomère HA acrylé sont lentement ajoutés à 100 ml d'eau déionisée et dissous dans celle-ci dans le cadre de la production d'hydrogels HA photoréticulés.

5. Procédé de fabrication d'une formulation d'hydrogel viscoélastique injectable comprenant de l'acide hyaluronique photoréticulé selon la revendication 1, dans lequel 0,05 à 1 % en poids de camphoquinone est ajouté au mélange d'AH et mélangé de manière homogène.

6. Procédé de fabrication d'une formulation d'hydrogel viscoélastique injectable comprenant de l'acide hyaluronique photoréticulé selon la revendication 1, dans lequel le mélange de camphoquinone et d'AH est exposé à la lumière visible pendant 30 à 240 minutes à température ambiante et laissé pour permettre la réaction de réticulation.

7. Procédé de fabrication d'une formulation d'hydrogel viscoélastique injectable comprenant de l'acide hyaluronique photoréticulé selon la revendication 1, dans lequel le gel est neutralisé après la réaction de réticulation avec 0,1 M d'acide chlorhydrique (HC1) dans une solution tampon phosphate (PBS) jusqu'à ce que le pH soit compris entre 6,8 et 7,4.
